# EUROPÄISCHE PATENTSCHRIFT

(11) **EP 1 117 639 B1**
(45) Veröffentlichungstag und Bekanntmachung des Hinweises auf die Patenterteilung: **15.12.2004**
(21) Anmeldenummer: 99946146.0
(22) Anmeldetag: 09.09.1999
(51) Int. Cl.: C07C 317/24, A01N 35/06, A01N 41/10

(54) **BENZOYLCYCLOHEXANDIONE, VERFAHREN ZU IHRER HERSTELLUNG UND IHRE VERWENDUNG ALS HERBIZIDE UND PFLANZENWACHSTUMSREGULATOREN**
BENZOYLCYCLOHEXANDIONES, METHOD FOR THE PRODUCTION AND USE THEREOF AS HERBICIDES AND PLANT GROWTH REGULATORS
PROCEDE DE PREPARATION ET D'UTILISATION DE BENZOYLCYCLOHEXANEDIONES EN TANT QU'HERBICIDES ET REGULATEURS DE LA CROISSANCE DE PLANTES

(30) Priorität: 10.10.1998 DE 19846792
(43) Veröffentlichungstag der Anmeldung: 25.07.2001
(73) Patentinhaber: Bayer CropScience GmbH, 65929 Frankfurt/Main (DE)
(72) Erfinder: VAN ALMSICK, Andreas, D-61440 Oberursel (DE); WILLMS, Lothar, D-65719 Hofheim (DE); AULER, Thomas, D-65812 Bad Soden a. Ts. (DE); BIERINGER, Hermann, D-65817 Eppstein (DE); ROSINGER, Christopher, D-65719 Hofheim (DE)
(86) Internationale Anmeldenummer: PCT/EP1999/006627
(87) Internationale Veröffentlichungsnummer: WO 2000/021924

(56) Entgegenhaltungen:
- WO-A-99/10327
- DE-A- 4 241 999
- US-A- 4 954 165
- US-A- 4 957 538
- PATENT ABSTRACTS OF JAPAN vol. 1996, no. 05, 31. Mai 1996 (1996-05-31) & JP 08 020554 A (HOKKO CHEM IND CO LTD), 23. Januar 1996 (1996-01-23)
- PATENT ABSTRACTS OF JAPAN vol. 1995, no. 11, 26. Dezember 1995 (1995-12-26) & JP 07 206808 A (HOKKO CHEM IND CO LTD), 8. August 1995 (1995-08-08)

## Beschreibung

Die Erfindung betrifft das technische Gebiet der Herbizide und Pflanzenwachstumsregutatoren, insbesondere das der Herbizide zur selektiven Bekämpfung von Unkräutern und Ungräsem in Nutzpflanzenkulturen.

Aus verschiedenen Schriften ist bereits bekannt, daß bestimmte Benzoylcyclohexandione, unter anderen auch solche, die in 3-Position des Phenylrings beispielsweise einen über eine Brücke verknüpften Rest tragen, herbizide Eigenschaften besitzen. So sind in JP-A 08 020554 solche Benzoylcyclohexandione beschrieben, die in der genannten Position einen substituierten Phenoxymethylrest tragen. JP-A 02 00222 beschreibt Benzoylcyclohexandione, die ebenfalls in der genannten 3-Position einen über eine Brücke verknüpften Rest tragen, wbei diese Brücke mindestens ein Atom aus der Gruppe Sauerstoff, Schwefel und Stickstoff enthält. WO 99/10327, WO 99/07688 und WO 99/03845 beschreiben Benzoylcyclohexandione, die in 3-Position einen über eine Kohlenstoffkette, die im Falle der WO 99/07688 auch durch Heteroatome unterbrochen ist, verknüpften heterocyclischen Rest tragen.

Die Anwendung der aus diesen Schriften bekannten Benzoylcyclohexandione ist jedoch häufig in der Praxis mit Nachteilen verbunden. So ist die herbizide oder pflanzenwachstumsregulierende Wirksamkeit der bekannten Verbindungen nicht immer ausreichend, oder bei ausreichender herbizider Wirksamkeit werden unerwünschte Schädigungen der Nutzpflanzen beobachtet.

Aufgabe der vorliegenden Erfindung ist die Bereitstellung von herbizid und pflanzenwachstumsregulierend wirksamen Verbindungen, die die aus dem Stand der Technik bekannten Nachteile überwinden.

Die Lösung der Aufgabe sind in 3-Position des Phenylrings speziell substituierte Benzoylcyclohexandione der allgemeinen Formel (I), 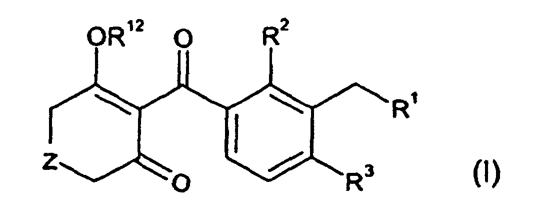 in der die Substituenten und Symbole die folgende Bedeutung haben: ,
- R¹: bedeutet Halogen-(C₁-C₆)-alkoxy, (C₃-C₈)-Cycloalkoxy, Aryl-(C₁-C₆)-alkoxy, 2-Tetrahydrofuranylmethoxy, 2-Tetrahydropyranylmethoxy, Ethoxyethoxyethoxy, Methoxyethoxyethoxy oder Cyclopropylmethoxy;
- R²: bedeutet Halogen;
- R³: bedeutet (C₁-C₆)-Alkylsulfonyl;
- R¹²: bedeutet Wasserstoff oder Benzoyl und
- Z: bedeutet CH₂, CH(CH₃) oder C(CH₃)₂.

Zahlreiche erfindungsgemäße Verbindungen der Formel (I) können in Abhängigkeit von äußeren Bedingungen, wie Lösungsmittel und pH-Wert, in unterschiedlichen tautomeren Strukturen auftreten.

Für den Fall, daß OR¹² für Hydroxy steht, sind folgende tautomeren Strukturen möglich:

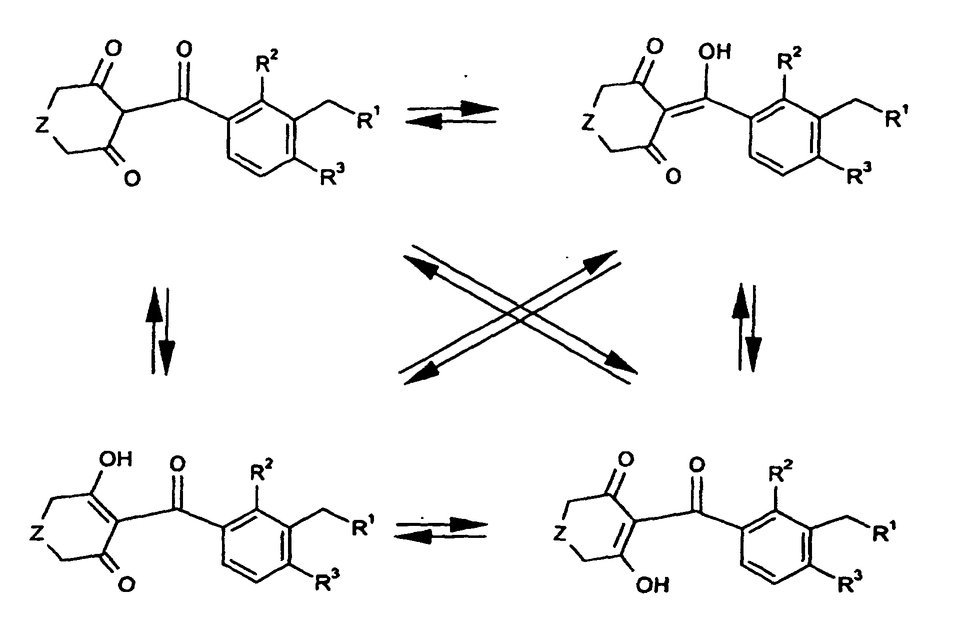

Je nach Art der Substituenten enthalten die Verbindungen der allgemeinen Formel (I) ein acides Proton, das durch Umsetzung mit einer Base entfernt werden kann. Als Basen eignen sich beispielsweise Alkalimetalle, wie Lithium, Natrium und Kalium, Erdalkalimetalle, wie Calcium und Magnesium, Ammoniak und organische Amine. Solche Salze sind ebenfalls Gegenstand der Erfindung.

In Formel (I) und allen nachfolgenden Formeln können kettenförmige kohlenstoffhaltige Reste wie Alkyl, Alkoxy, Halogenalkyl, Halogenalkoxyjeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 4 C-Atomen, bevorzugt. Alkylreste, auch in den zusammengesetzten Bedeutungen wie Alkoxy, Halogenalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl.

Cycloalkyl bedeutet ein carbocyclisches, gesättigtes Ringsystem mit drei bis acht C-Atomen, z.B. Cyclopropyl, Cyclopentyl oder Cyclohexyl.

Halogen bedeutet Fluor, Chlor, Brom oder lod. Halogenalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Halogenalkoxy ist z.B. OCF₃, OCHF₂, OCH₂F, CF₃CF₂O, OCH₂CF₃ und OCH₂CH₂Cl; entsprechendes gilt für Halogenalkenyl und andere durch Halogen substituierte Reste.

Aryl steht für einen aromatischen mono- oder polycyclischen Kohlenwasserstoffrest, z.B. Phenyl, Naphthyl, Biphenyl und Phenanthryl.

Die Verbindungen der allgemeinen Formel I können je nach Art und Verknüpfung der Substituenten als Stereoisomere vorliegen. Sind beispielsweise eine oder mehrere Alkenylgruppen vorhanden, so können Diastereomere auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel I umfaßt, jedoch nicht spezifisch definiert sind.

Bevorzugt sind Verbindungen der allgemeinen Formel (I), worin
- R¹: bedeutet Halogen-(C₁-C₆)-alkoxy, (C₃-C₈)-Cycloalkoxy, Benzylethoxy, 2-Tetrahydrofuranylmethoxy, 2-Tetrahydropyranylmethoxy, Ethoxyethoxyethoxy, Methoxyethoxyethoxy oder Cyclopropylmethoxy;
- R²: bedeutet Chlor,
- R³: bedeutet Methylsulfonyl;
- R¹²: bedeutet Wasserstoff, und
- Z: bedeutet CH₂.

Besonders bevorzugt sind Verbindungen der allgemeinen Formel (I), worin
R¹ 2,2,2-Trifluorethoxy, 2,2-Difluorethoxy, 3,3,3,2,2-Pentafluorpropoxy, Cyclopentoxy, Cyclobutyloxy, Cyclopropylmethoxy, Benzylethoxy, 2-Tetrahydrofuranylmethoxy, 2-Tetrahydropyranylmethoxy, Ethoxyethoxyethoxy, Methoxyethoxyethoxy oder Cyclopropylmethoxy bedeutet.

Ganz besonders bevorzugt sind Verbindungen der allgemeinen Formel (I), worin
R¹ 2,2,2-Trifluorethoxy, 2,2-Difluorethoxy, 3,3,3,2,2-Pentafluorpropoxy, Benzylethoxy, 2-Tetrahydrofuranylmethoxy, 2-Tetrahydropyranylmethoxy, Ethoxyethoxyethoxy oder Methoxyethoxyethoxy bedeutet.

In allen nachfolgend genannten Formeln haben die Substituenten und Symbole, sofern nicht anders definiert, dieselbe Bedeutung wie unter Formel (I) beschrieben.

Die erfindungsgemäßen Verbindungen können je nach Bedeutung der Substituenten beispielsweise nach einem oder mehreren der in den folgenden Schemata angegebenen Verfahren hergestellt werden.

Durch die in Schema 1 angegebene Umsetzung einer Verbindung der Formel (II) mit einer Verbindung der Formel (III), in der R für Hydroxy, Chlor, Brom oder Cyano steht, erhält man erfindungsgemäße Verbindungen der Formel (I). Dazu wird die Verbindung der Formel (11) im Fall von R = Hydroxy in Gegenwart wasserentziehender Mittel, wie DCC, oder im Fall von R = Chlor oder Brom basenkatalysiert und in Anwesenheit einer Cyanid-Quelle, oder im Fall von R = Cyano basenkatalysiert direkt mit (III) umgesetzt. Diese Methoden sind beispielsweise in EP-A 0 369 803 und EP-B 0 283 261 beschrieben.

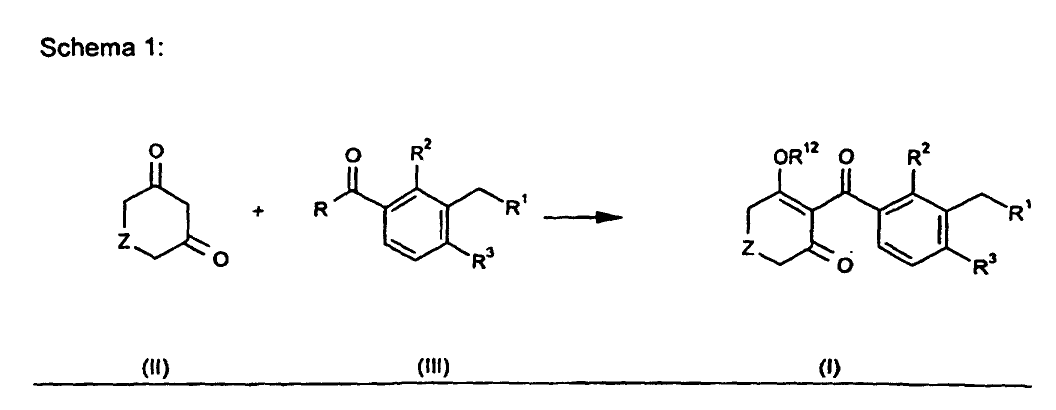

Die Dicarbonylverbindungen der Formel (II) sind entweder kommerziell erhältlich oder können gemäß bekannten Methoden hergestellt werden. Solche Methoden sind beispielsweise aus EP-A 0 283 261, *Tetrahedron Lett*. **32**, 3063 (1991), *J. Org. Chem*. **42**, 2718, (1977), *Helv*. *Chim. Acta.* **75**, 2265 (1992), *Tetrahedron Lett*. **28**, 551 (1987). *Tetrahedron Lett*. **32**, 6011 (1991, *Chem*. *Lett*. 551, **1981**, *Heterocycles* 26,2611 (1987) bekannt.

Verbindungen oben genannter Formel (III) können gemäß bekannten Methoden aus Verbindungen der Formel (III), in der R für Hydroxy oder Alkoxy steht, hergestellt werden.

Verbindungen der Formel (III), in der R¹ für Alkoxy steht, können beispielsweise gemäß Schema 2 aus Verbindungen der Formel (IV), in der Hal für Halogen steht, hergestellt werden. 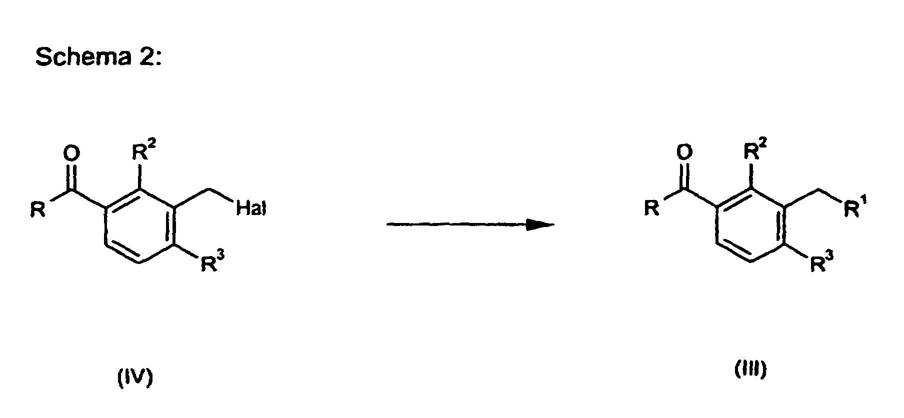
2.1 Vebindungen der Formel (III) sind erhältlich durch basenkatalysierte Umsetzung mit Verbindungen R¹-H, wie Alkohole, Thioalkohole, Amide, Amine, Heteroaromaten, Heterocyclen. Solche Reaktionen sind beispielsweise bekannt aus *J. C. Chem. Res., Synop.* **1994**, 174, *Tetrahedron Lett*. **27**, 279 (1986, *J. Org. Chem.* **55**, 6037 (1990), *J. Org. Chem.* **54**, 3757 (1989).
2.2 Vebindungen der Formel (III) sind ebenfalls erhältlich durch Umsetzung mit lithiumorganischen Verbindungen der Formel R¹-Li. Solche Reaktionen sind beispielsweise bekannt aus *Synth. Commun.* **18**, 1035, (1988), *J. Org. Chem*. **46**, 3132 (1981).

Verbindungen der Formel (III) sind auch gemäß Schema 3 erhältlich durch basenkatalysierte Umsetzung einer Verbindung der Formel (V), in der Z¹ für OH, SH, NH-Alkyl, NH-Aryl oder NH-Heteroaryl steht, mit kommerziell erhältlichen oder gemäß bekannten Methoden herstellbaren Verbindungen der Formel R¹-Z², in der Z² für eine Abgangsgruppe steht, wie Halogen, Phenoxy und Alkylsulfonyl. Solche Reaktionen sind beispielsweise bekannt aus *Synthesis* **1980,** 573, *Tetrahedron Lett*. **37**, 4065 (1996).

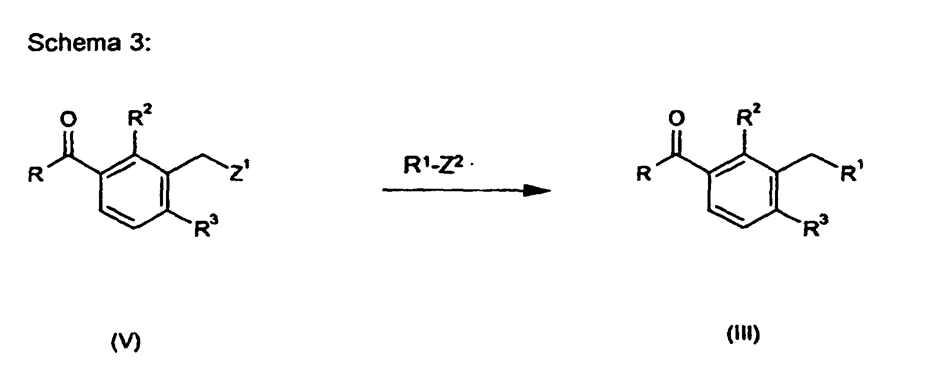

Die in Schema 4 angegebene Umsetzung einer Verbindung der Formel (la) mit einem Halogenierungsreagenz, wie Oxalylchlorid oder Oxalylbromid, führt zu erfindungsgemäßen Verbindungen der Formel (Ib), die durch Reaktion, gegebenenfalls unter Basenkatalyse, mit Nukleophilenumgesetzt werden können. Solche Reaktionen sind beispielsweise beschrieben in *Synthesis* **12**, 1287 (1992).

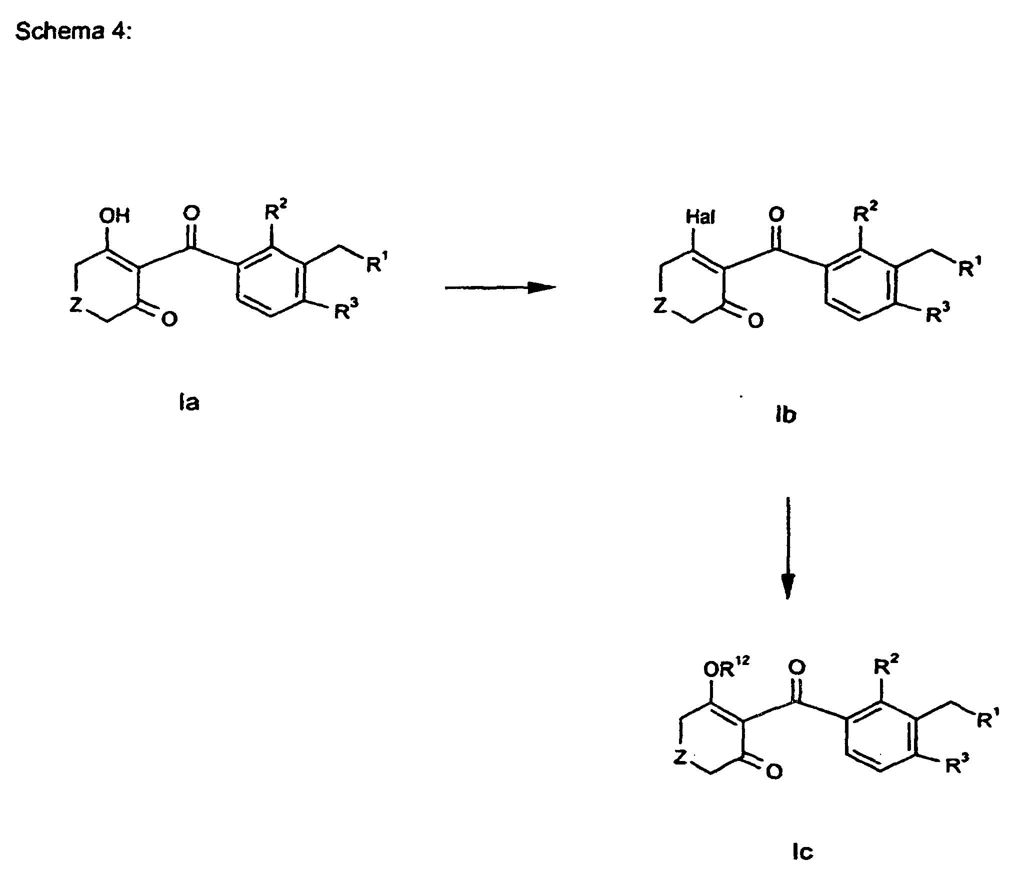

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert . werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauemde Cyperusarten gut erfaßt.
Bei dikotylen Unkrautarten erstreckt sich das Wirkungsspektrum auf Arten wie z.B.
Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutem.
Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft.
Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.
Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Emtegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Altemativ können neue Pflanzen mit veränderten Eigenschaften mit Hilfe gentechnisctier Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996)423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Mofeküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 96-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streuund Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshitfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührem, Kolloidmühlen und/oder statischen Mischem unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%. Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlomitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenit; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester, propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vemolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvem, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Die nachstehenden Beispiele erläutern die Erfindung.

### A. Chemische Beispiele

### 1. Herstellung von 2-(2-Chlor-3-cyclohexanyloxymethyl-4-methylsulfonylbenzoyl)-cyclohexan-1,3-dion

### Schritt 1: 2-Chlor-6-methylthiotoluol

200 g (1.24 mol) 2,6-Dichlortoluol wurden in 600 ml Hexamethylphosphorsäuretriamid gelöst und mit 130.41 g (1,86 mol) Natriumthiomethylat versetzt. Anschließend wurde für 3 h auf 100 °C erhitzt. Man ließ abkühlen, fügte 88.2 g (0.5 mol) Jodmethan hinzu und ließ 0,5 h bei Raumtemperatur nachrühren. Anschließend wurde der Ansatz auf 3.5 l Wasser gegeben und mit Essigester extrahiert. Die vereinigten organischen Phasen wurden mit Wasser gewaschen, über MgSO₄ getrocknet und am Rotationsverdampfer vollständig eingeengt.
Ausbeute: 208.85 g (97 % der Theorie), gelbes Öl
¹H NMR (CDCl₃): δ 2.4 (s,3H), 2.42 (s,3H), 7.0-7.18 (m,3H)

### Schritt 2: 2-Chlor-3-methyl-4-methylthio-acetophenon

47.36 g (0.60 mol) Acetylchlorid in 200 ml 1,2-Dichlorethan wurden bei 15-20 °C zu einer Suspension von 90.79 g (0.68 Mol) Aluminiumchlorid in 200 ml 1,2-Dichlorethan getropft. Anschließend wurde eine Lösung von 103.14 g (0.60 mol) 2-Chlor-6-methylthiotoluol in 400 ml 1,2-Dichlorethan zugetropft. Man ließ über Nacht bei Raumtemperatur rühren und gab das Reaktionsgemisch auf eine Mischung von 1 l Eis und 300 ml konz. Salzsäure. Es wurde mit Methylenchlorid extrahiert. Die vereinigten organischen Phasen wurden mit Wasser gewaschen, über MgSO₄ getrocknet und am Rotationsverdampfer vollständig eingeengt. Der Rückstand wurde im Vakuum destilliert.
Ausbeute: 111.24 g (87 % der Theorie), farblose Kristalle, Fp.: 45.5-46 °C
¹H NMR (CDCl₃): δ 2.42 (s,3H), 2.5 (s,3H), 2.6 (s,3H), 7.05 (d,1 H), 7.35 (d,1H)

### Schritt 3: 2-Chlor-3-methyl-4-methylsulfonyl-acetophenon

223.48 g (1.04 mol) 2-Chlor-3-methyl-4-methylthio-acetophenon wurden in 1.8 l Eisessig gelöst und mit 27.47 g (0.08 mol) Natriumwolframat versetzt. Anschließend ließ man unter Kühlung 203.83 g einer 30 %igen Wasserstoffperoxidlösung zutropfen und 1.5 d bei Raumtemperatur nachrühren. Es wurde mit 1.5 I Wasser verdünnt, der ausgefallenen Festtsoff abgesaugt und mit Wasser gewaschen und getrocknet.
Ausbeute: 123.35 g (48 % der Theorie), farblose Kristalle, Fp.: 110-111 °C
¹H NMR (CDCl₃): δ 2.62 (s,3H), 2.8 (s,3H), 3.12 (s,3H), 7.38 (d, 1H), 8.08 (d, 1H)

### Schritt 4: 2-Chlor-3-methyl-4-methylsulfonyl-benzoesäure

60 g (0,24 mol) 2-Chlor-3-methyl-4-methylsulfonyl-acetophenon wurden in 510 ml Dioxan gelöst und mit 870 g 13 %ige Natriumhypochloritiösung versetzt. Anschließend wurde noch 1 h auf 80 °C erwärmt. Nachdem Abkühlen wurde die untere Phase abgetrennt, mit Wasser verdünnt und mit Salzsäure angesäuert. Der ausgefallenen Feststoff wurde abgesaugt, mit Wasser gewaschen und getrocknet.
Ausbeute: 53.02 g (88 % der Theorie), farblose Kristalle, Fp.:, 230-231 °C
¹H NMR (Me₂SO-*d6*): δ 2.75 (s,3H), 3.3 (s,3H), 7.75 (d, 1H), 7.98 (d, 1H)

### Schritt 5: Methyl 2-Chlor-3-methyl-4-methylsulfonyl-benzoat

53.02 g (0.21 mol) 2-Chlor-3-methyl-4-methylsulfonyl-benzoesäure wurden in 400 ml Methanol gelöst und bei Rückflußtemperatur 3 h mit HCl begast. Anschließend ließ man abkühlen und engte am Roatationsverdampfer vollständig ein.
Ausbeute: 54.93 g (98 % der Theorie), farblose Kristalle, Fp.: 107-108 °C
¹H NMR (CDCl₃): δ 2.82 (s,3H), 3.15 (s,3H), 3.98 (s,3H), 7.65 (d,1H), 8.04 (d, 1H)

### Schritt 6: Methyl 3-brommethyl-2-chior-4-methylsulfonyl-benzoat

44.14 g (0.17 mol) Methyl 2-Chlor-3-methyl-4-methylsulfonyl-benzoat wurden in 600 ml Tetrachlorkohlenstoff gelöst mit 29.91 g (0.17 mol) N-Bromsuccinimid und mit 0.41 g Dibenzoylperoxid versetzt. Anschließend ließ man unter Rückfluß kochen und mit einer 300 W Lampe belichten. Das Reaktionsgemisch wurde filtriert, das Filtrat eingeengt und der Rückstand in Diethylether aufgenommen. Die Lösung wurde mit n-Heptan versetzt, der ausgefallene Feststoff abgesugt und getrocknet.
Ausbeute: 38.82 g (67 % der Theorie), farblose Kristalle, Fp.: 74-75 °C
¹H NMR (CDCl₃): δ 3.35 (s,3H), 4.00 (s,3H), 5.3 (s,br, 2H), 7.8 (d,1H), 8.15 (d,1H)

### Schritt 7: 2-Chlor-3-cyclohexanyloxymethyl-4-methylsulfonyl-benzoesäure

1.0 g (2.93 mmol) Methyl 3-brommethyl-2-chlor-4-methylsulfonyl-benzoat wurde in 10 ml Cyclohexanol gelöst und mit 0.33 g (2.93 mmol) Kalium-tert-butylat versetzt. Man ließ über Nacht bei Raumtemperatur rühren und engte anschließend am Rotationsverdampfer ein. Der Rückstand wurde in 16 ml Tetrahydrofuran und 8 ml Wasser gelöst und zusammen mit 0.55 g NaOH (13.74 mmol) 4 h unter Rückfluß gekocht. Anschließend ließ man abkühlen, engte am Rotationsverdampfer weitgehend ein und versetzte anschließend den wässrigen Rückstand mit 2 M HCl. Anschließend wurde mit Methylenchlorid extrahiert, die vereinigte organische Phase über MgSO₄ getrocknet und am Rotationsverdampfer vollständig eingeengt.
Ausbeute: 0.53 g (52 % der Theorie), farbloses Öl
¹H NMR (CDCl₃): δ 0.9 (m,6H), 1,3 (m,4 H), 3.3 (s,3H), 4.75 (m,1H), 5:3 (s,2H), 7.9 (d,1H), 8.1 (d,1H)

### Schritt 8: 3-Oxo-1-cyclohexenyl 2-chlor-3-cyclohexanyloxymethyl-4-methylsulfonyl-benzoat

0.53 g (1.53 mmot) 2-Chlor-3-cyclohexanyloxymethyl-4-methylsulfonyl-benzoesäure wurden in 23 ml Methylenchlorid mit 2 Tropfen N,N-Dimethylformamid und 0.59 g (4.58 mmol) Oxalylchlorid versetzt und 2.5 h unter Rückfluß gekocht. Anschließend wurde am Rotationsverdampfer abgezogen, der Rückstand in 23 ml Methylenchlorid aufgenommen und bei 0 °C mit 0.19 g (1.68 mmol) Cyclohexandion und 0.46 g (4.58 mmol) Triethylamin versetzt. Man ließ 4 h bei Raumtemperatur nachrühren.. Anschließend wurde am Rotationsverdampfer eingeengt und chromatographisch gereinigt (Kieselgel, Essigester:Hexan = 1:1).
Ausbeute: 0.1 g (15 % der Theorie), farbloses Öl
¹H NMR (CDCl₃): δ 0.9 (m,6H), 1.3 (m,4H), 2.35 (m,2H), 2.5 (m,2H), 2.7 (m,2H), 3.35 (s,3H), 5.4 (s,br, 2H), 6.1 (s, 1H), 7.95 (d,2H), 8.2 (d,2H)

### Schritt 9: 2-(2-Chlor-3-cyclohexanyloxymethyl-4-methylsulfonyl-benzoyl)-cyclohexan-1,3-dion

0.10 g (0.23 mmol) 3-Oxo-1-cyclohexenyl 2-chlor-3-cyclohexanyloxymethyl-4-methylsulfonyl-benzoat, 1 Tropfen Acetoncyanhydrin und 0.04 g (0.39 mMol) Triethylamin wurden in 5 ml Acetonitril gelöst und über Nacht bei Raumtemperatur gerührt. Anschließend wurde am Rotationsverdampfer eingeengt, mit 5 ml Wasser versetzt und mit 5 M HCl sauer gestellt. Es wurde mit Essigester extrahiert, mit Wasser gewaschen, über MgSO₄ getrocknet und am Rotationsverdampfer vollständig eingeengt.
Ausbeute: 0.1 g (100 % der Theorie), farbloses Öl, R_{f} = 0.07 (SiO₂/Essigester)
¹H NMR (CDCl₃): δ 0.9 (m,6H), 1.3 (m,4H), 2.1 (m,2H), 2.45 (m,2H), 2.85 (m,2H), 3.3 (s,3H), 4.55 (s,1H), 5.35 (s,br, 2H), 7.3 (d,2H), 8.15 (d,2H)

### 2. Herstellung von 2-(2-Chlor-4-methylsulfonyl-3-phenoxymethyl-benzoyl)-cyclohexan-1,3-dion

### Schritt 1: 2-Chlor-4-methylsulfonyl-3-phenoxymethyl-benzoesäure

1.0 g (2.93 mmol) Methyl 3-brommethyl-2-chlor-4-methylsulfonyl-benzoat und 0.28 g (2.93 mmol) Phenol wurden in 20 ml Dimethylformamid gelöst und mit 0.14 g (3.51 mmol) 60 %iges Natriumhydrid versetzt. Man ließ über Nacht bei Raumtemperatur rühren und engte anschließend im Hochvakuum am Rotationsverdampfer ein. Der Rückstand wurde in 16 ml Tetrahydrofuran und 8 ml Wasser gelöst und zusammen mit 0.23 g NaOH (5.85 mmol) 4 h unter Rückfluß gekocht. Anschließend ließ man abkühlen, engte am Rotationsverdampfer weitgehend ein und versetzte anschließend den wässrigen Rückstand mit 2 M HCl. Anschließend wurde mit Methylenchlorid extrahiert, die vereinigte organische Phase über MgSO₄ getrocknet und am Rotationsverdampfer vollständig eingeengt
Ausbeute: 0.67 g (67 % der Theorie), farbloses Öl
¹H NMR (Me₂SO-*d6*): δ 3.3 (s,3H), 5.55 (s,2H), 6.98-7.05 (m,3H), 7.35 (m,2H), 7.95 (d, 1H), 8.1 (d,1 H)

### Schritt 2: 3-Oxo-1-cyclohexenyl 2-chlor-4-methylsulfonyl-3-phenoxymethylbenzoesäure

0.67 g (1.97 mmol) 2-Chlor-4-methylsulfonyl-3-phenoxymethyl-benzoesäure wurden in 30 ml Methylenchlorid mit 2 Tropfen N, N-Dimethylformamid und 0.76 g (5.9 mmol) Oxalylchlorid versetzt und 2.5 h unter Rückfluß gekocht. Anschließend wurde am Rotationsverdampfer abgezogen, der Rückstand in 30 ml Methylenchlorid aufgenommen und bei 0 °C mit 0.24 g (2.16 mmol) Cyclohexandion und 0.60 g (5.9 mmol) Triethylamin versetzt. Man ließ 4 h bei Raumtemperatur nachrühren. Anschließend wurde am Rotationsverdampfer eingeengt und chromatographisch gereinigt (Kieselgel, Essigester:Hexan =1:1).
Ausbeute: 0.51 g (60 % der Theorie), farbloses Öl
¹H NMR (CDCl₃): δ 2.15 (m,2H), 2.45 (m,2H), 2.7 (m,2H), 3.2 (s,3H), 5.75 (s,2H), 6.08 (s, 1H), 7.0-7.1 (m,3H), 7.35 (m,2H), 7.98 (d,1H), 8.25 (d, 1H)

### Schritt 3: 2-(2-Chlor-4-methylsulfonyl-3-phenoxymethyl-benzoyl)-cyclohexan-1,3-dion

0.51 g (1.17 mmol) 3-Oxo-1-cyclohexenyl 2-chlor-4-methylsulfonyl-3-phenoxymethylbenzoesäure, 1 Tropfen Acetoncyanhydrin und 0.21 g (2.04 mMol) Triethylamin wurden in 20 ml Acetonitril gelöst und über Nacht bei Raumtemperatur gerührt. Anschließend wurde am Rotationsverdampfer eingeengt, mit 5 ml Wasser versetzt und mit 5 M HCl sauer gestellt. Es wurde mit Essigester extrahiert, mit Wasser gewaschen, über MgSO₄ getrocknet und am Rotationsverdampfer vollständig eingeengt. Es wurde mit Essigester extrahiert, mit Wasser gewaschen, über MgSO₄ getrocknet und am Rotationsverdampfer vollständig eingeengt.
Ausbeute: 0.5 g (98 % der Theorie), farbloses Öl, R_{f} = 0.22 (SiO₂/Essigester)
¹H NMR (CDCl₃): δ 2.08 (m,2H), 2.45 (m,2H), 2.85 (m,2H), 3.2 (s,3H), 5.7 (s,br, 2H), 7.0 (d,2H), 7.05 (m,2H), 7.35 (m,3H), 8.18 (d,2H).

Die in nachfolgenden Tabellen aufgeführten Beispiele wurden analog oben genannten Methoden hergestellt beziehungsweise sind analog oben genannten Methoden erhältlich.

Die in den Tabellen verwendeten Abkürzungen bedeuten:

| | | |
|---|---|---|
| Ac = Acetyl | Bu = Butyl | Bz = Benzoyl |
| Et = Ethyl | Me = Methyl | Pr = Propyl |
| c = cyclo | d = Dublett | m = Multiplett |
| s = Singulett | t = Triplett | i = iso |
| Fp. = Festpunkt | dd = Doppelduplett | |

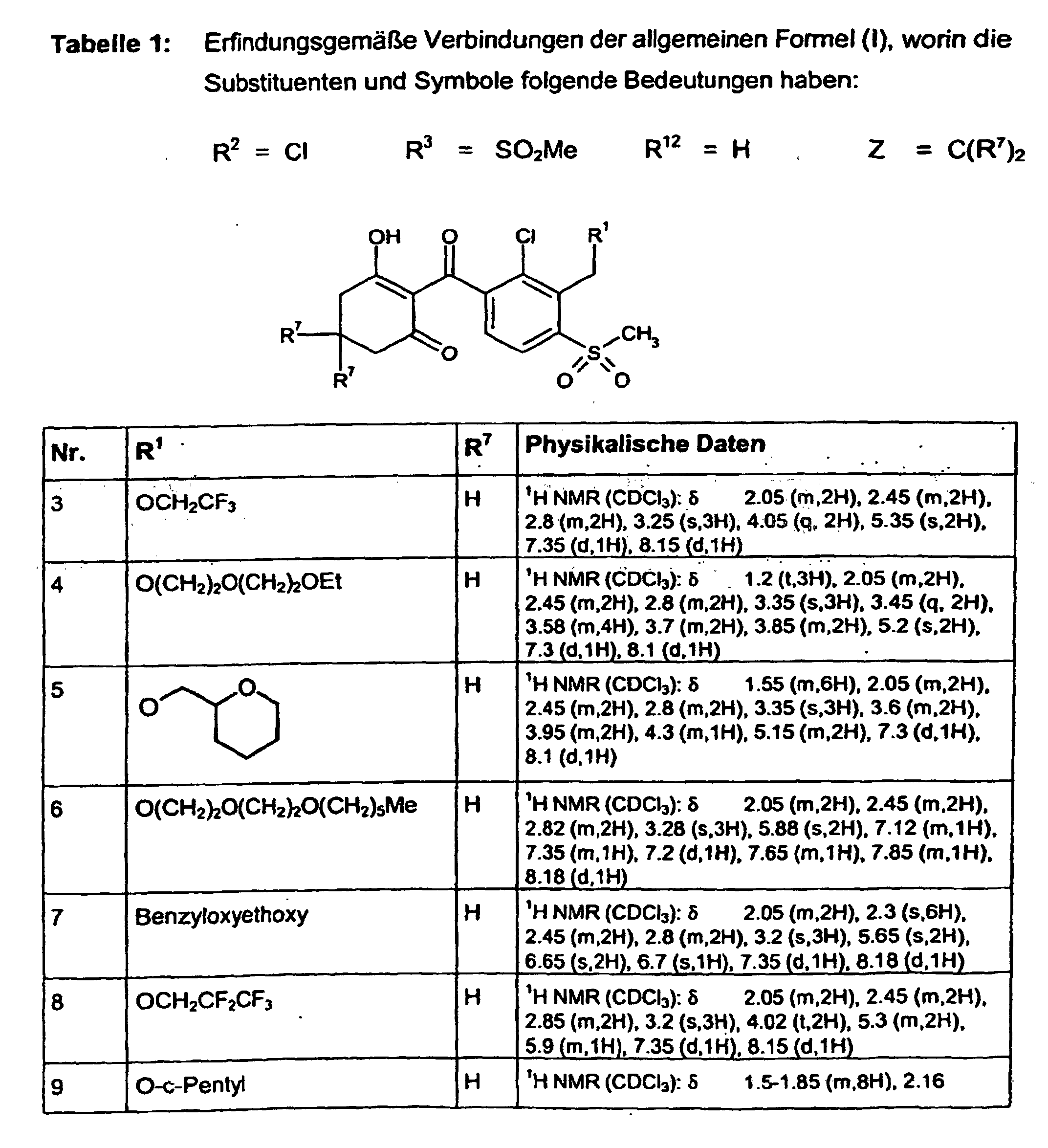

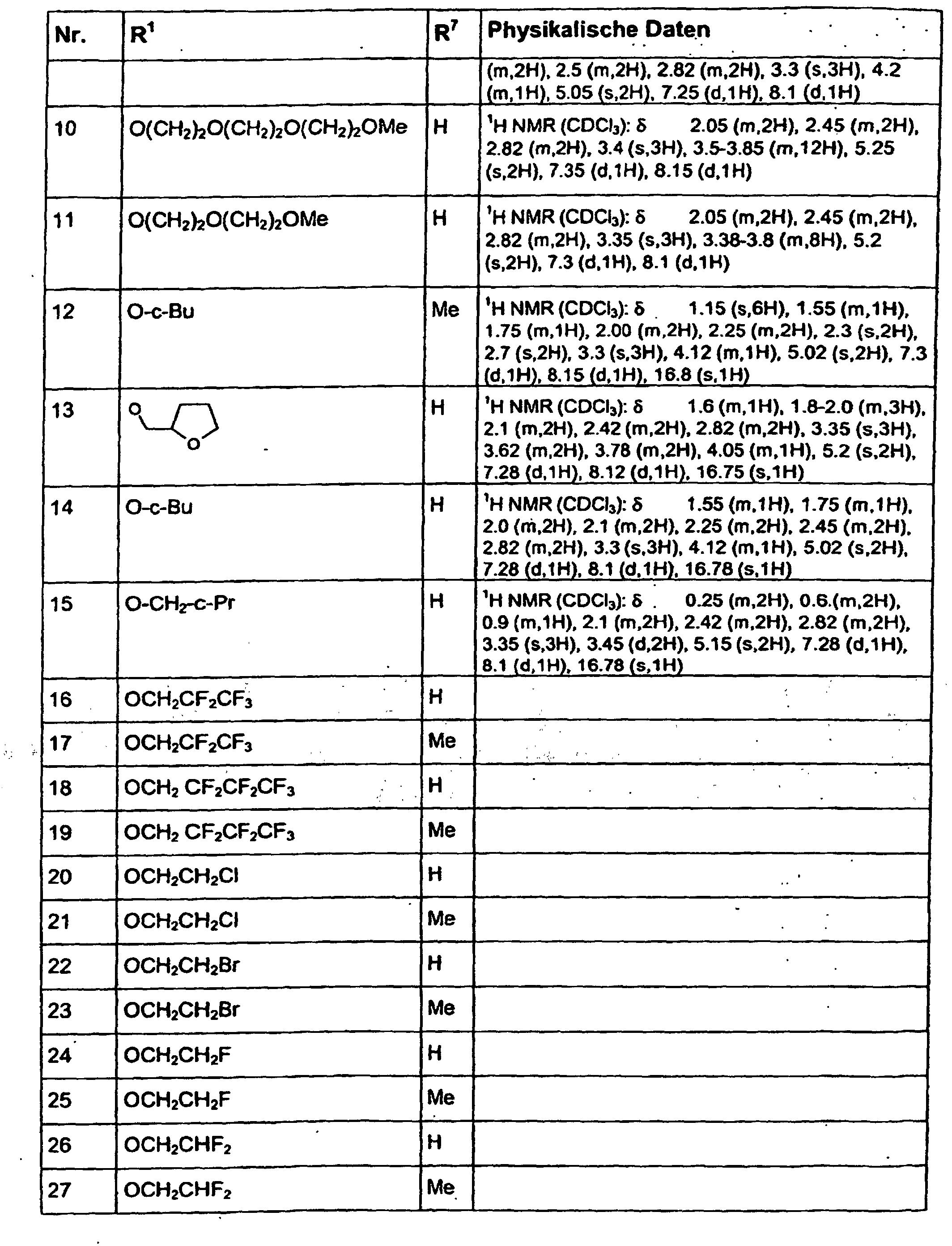

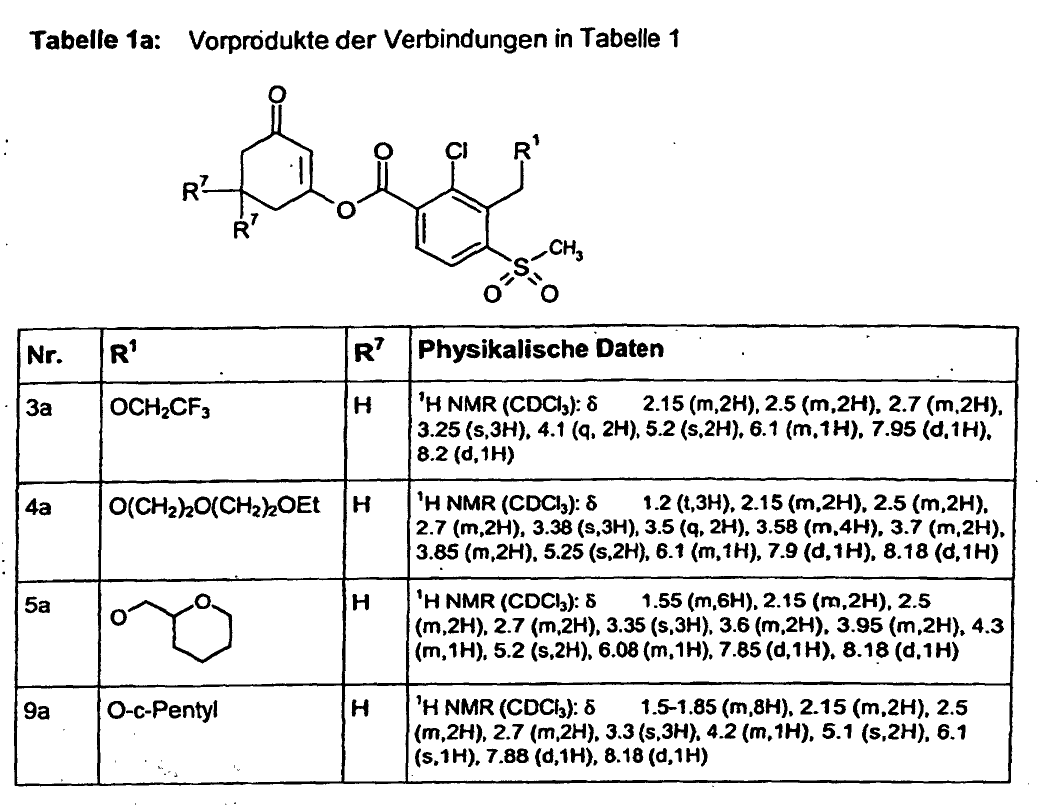

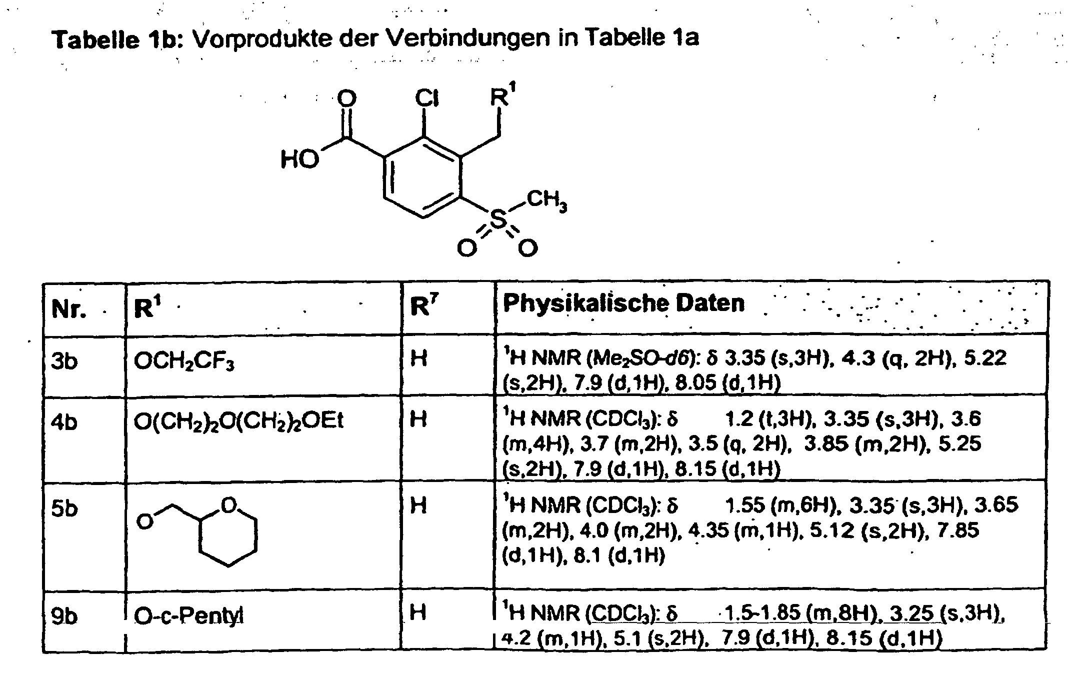

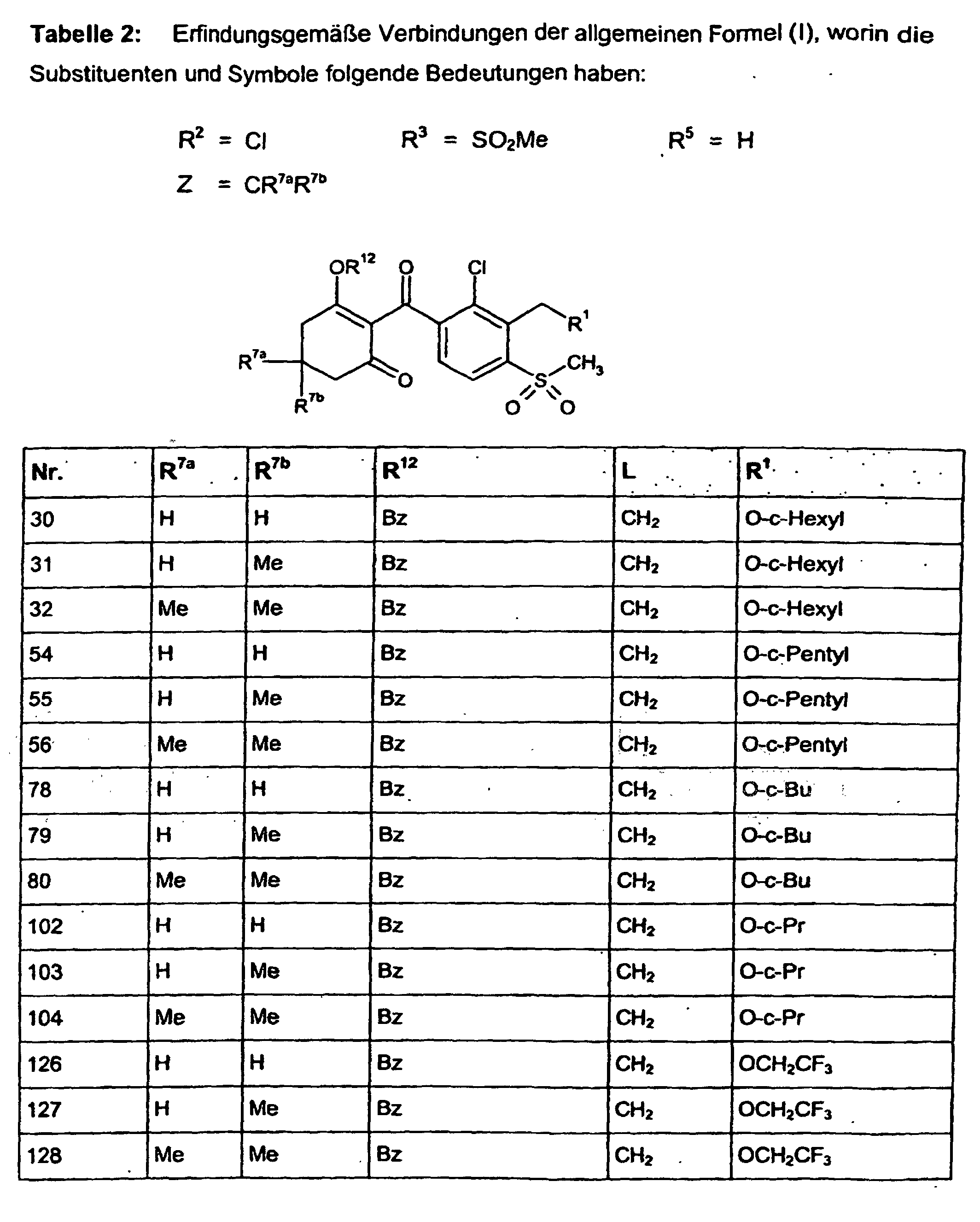

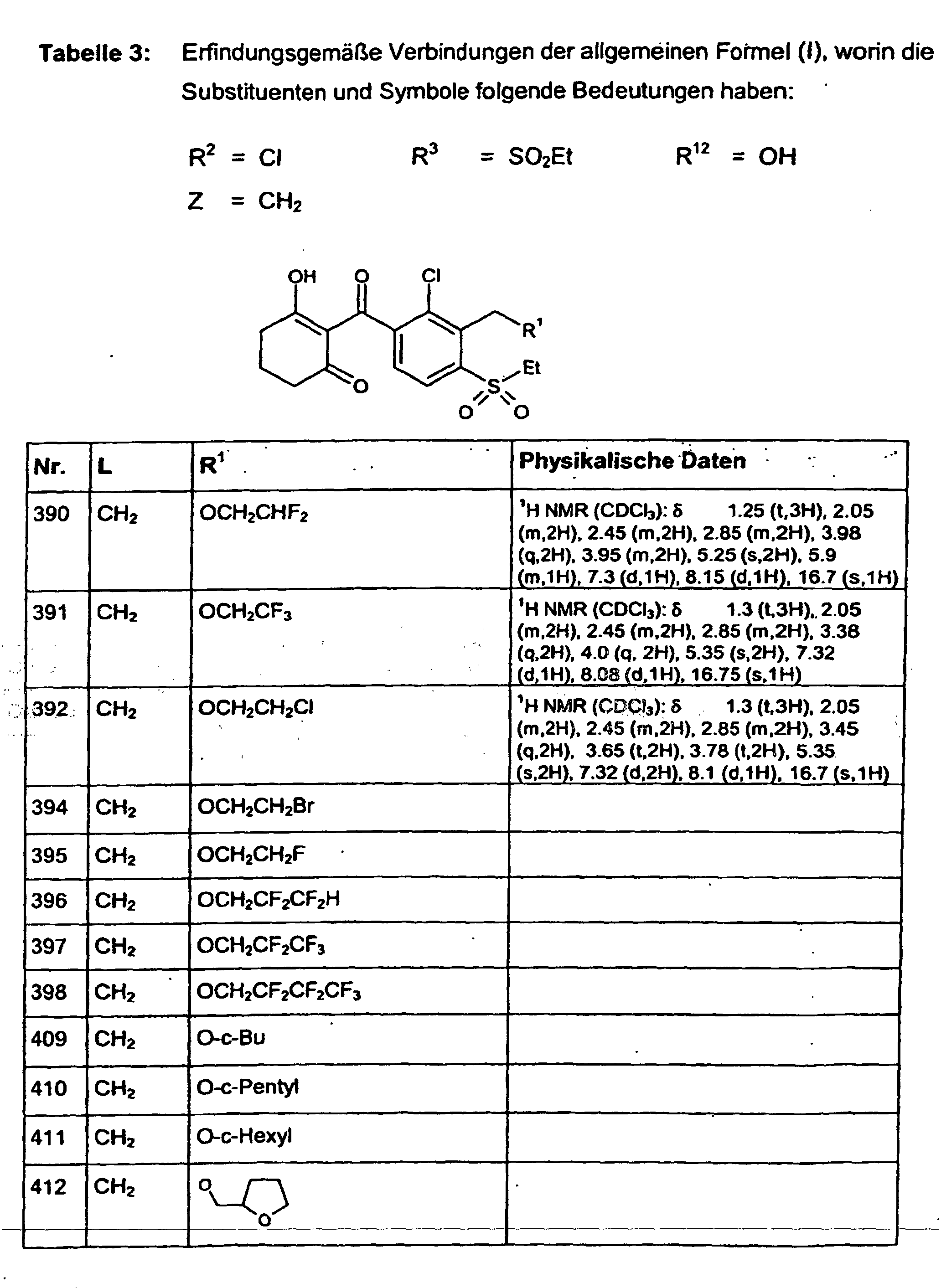

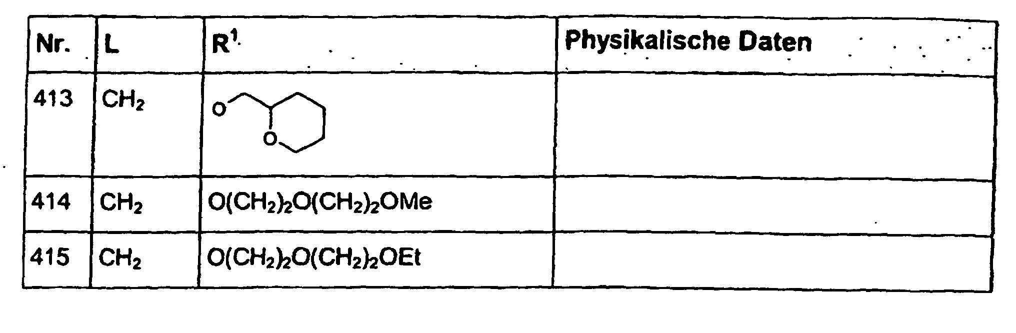

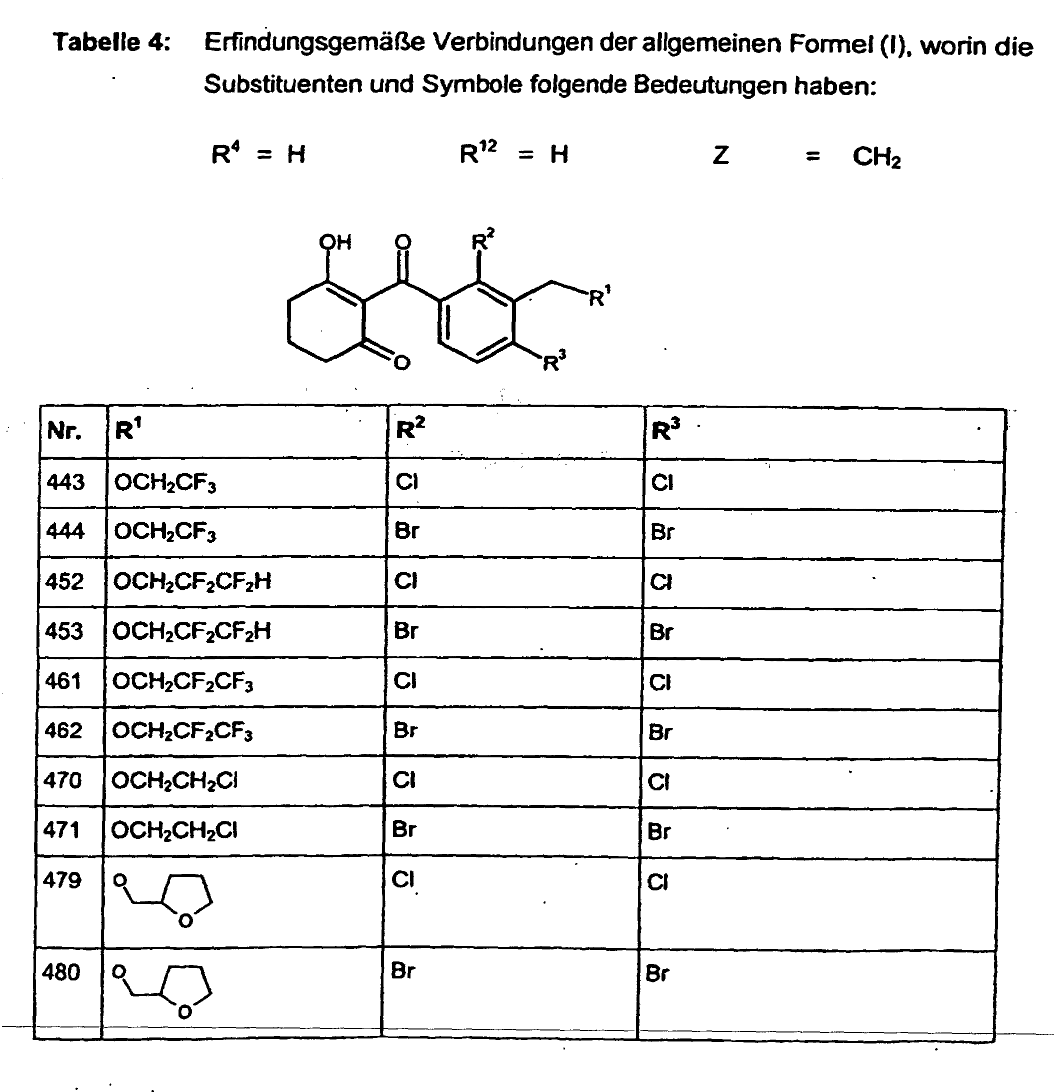

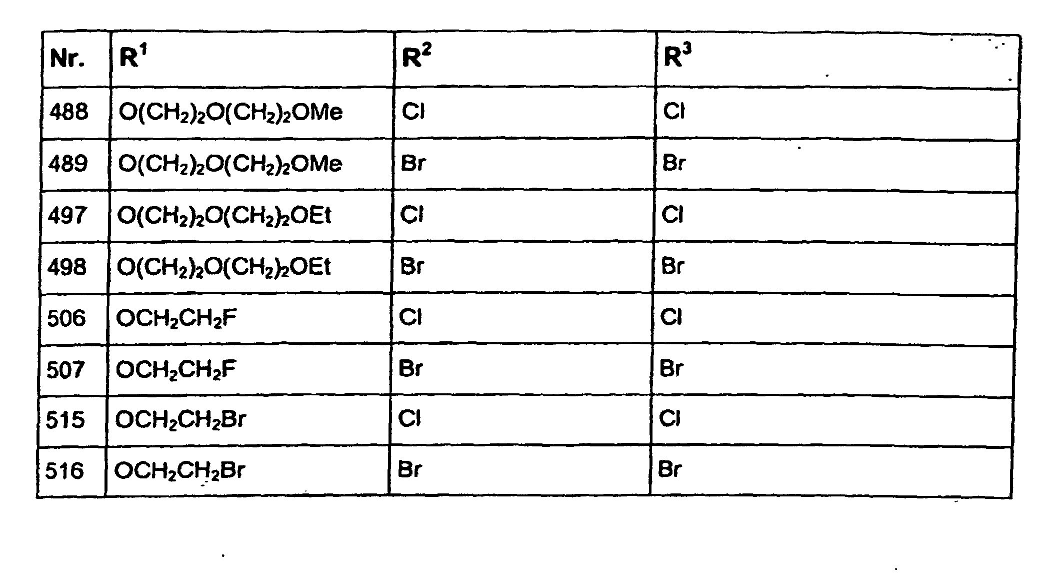

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teit oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | |
|---|---|
| 75 Gew.-Teile | einer Verbindung der allgemeinen Formel(I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 Gew.-Teile | einer Verbindung der allgemeinen Formel (I), |
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen von mono- und dikotylen Unkrautpflanzen werden in Papptöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in einer Dosierung von umgerechnet 1 kg Aktivsubstanz oder weniger pro Hektar auf die Oberfläche der Abdeckerde appliziert. Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Dabei zeigen beispielsweise die Verbindungen der Beispiele Nr. 5 und 19 eine mindestens 80 %ige Wirkung gegen Stellaria media, Lolium multiflorum und Amaranthus retroflexus. Die Verbindungen der Beispiele Nr. 2 und 8 zeigen eine mindestens 90 %ige Wirkung gegen Amaranthus retroflexus, Stellaria media und Setaria viridis. Die Verbindungen der Beispiele Nr. 2 und 18 zeigen eine 100 %ige Wirkung gegen Amaranthus retroflexus und Sinapis arvensis.

### 2. Unkrauktwirkung im Nachauflauf

Samen von mono- und dikotylen Unkräutern werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden in einer Dosierung von umgerechnet 1 kg Aktivsubstanz oder weniger pro Hektar mit einer Wasseraufwandmenge von umgerechnet 600 bis 800 l/ha auf die grünen Pflanzenteile gesprüht. Nach 3 bis 4 Wochen Standzeit der Versuchspflanzen im Gewächshaus unter optimalen Wachstumsbedingungen wird die Wirkung der Präparate im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise zeigen die Verbindungen der Beispiele Nr. 20, 32 und 18 eine mindestens 80 %ige Wirkung gegen Sinapis arvensis und Amaranthus retroflexus. Die Verbindungen der Beispiele Nr. 2 und 20 zeigen eine mindestens 80 %ige Wirkung gegen Stellaria media und Amaranthus retroflexus. Die Verbindungen der Beispiele Nr. 2 und 18 zeigen eine mindestens 90 %ige Wirkung gegen Sinapis arvensis und Stellaria media.

### 3. Wirkung auf Schadpflanzen in Reis

Typische Schadpflanzen in Reiskulturen werden im Gewächshaus unter Paddyreis-Bedingungen (Anstauhöhe des Wassers: 2 - 3 cm) angezogen. Nach der Behandlung mit den formulierten erfindungsgemäßen Verbindungen in einer Dosierung von umgerechnet 1 kg Aktivsubstanz oder weniger pro Hektar werden die Versuchspflanzen im Gewächshaus unter optimalen Wachstumsbedingungen aufgestellt und während der gesamten Versuchszeit so gehalten. Etwa drei Wochen nach der Applikation erfolgt die Auswertung mittels optischer Bonitur der Pflanzenschäden im Vergleich zu unbehandelten Kontrollen. Die erfindungsgemäßen Verbindungen weisen sehr gute herbizide Wirkung gegen Schadpflanzen auf. Dabei zeigen beispielsweise die Verbindungen der Beispiele Nr. 2, 8 und 32 eine mindestens 80 %ige Wirkung gegen Cyperus iria und Echinocloa crus-galli.

### 4. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja und Zuckerrüben im Vor- und Nachauflaufverfahren in der Regel selbst bei hohen Wirkstoffdosierungen ungeschädigt oder nahezu ungeschädigt lassen. Einige Substanzen schonen darüber hinaus auch Gramineen-Kulturen wie z.B. Gerste, Weizen und Reis. Die Verbindungen der Formel (I) zeigen teilsweise eine hohe Selektivität und eignen sich deshalb zur Bekämpfung von unerwünschten Pflanzenwuchs in landwirtschaftlichen Kulturen.

## Patentansprüche

1. Benzoylcyclohexandione der allgemeinen Formel (I), 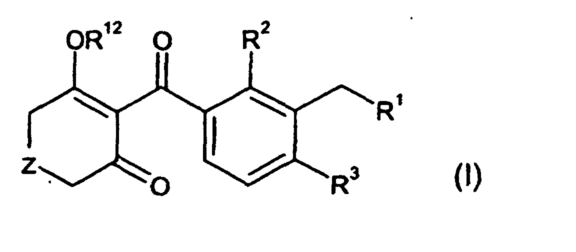 worin
R¹ bedeutet Halogen-(C₁-C₆)-alkoxy, (C₃-C₈)-Cycloalkoxy, Aryl-(C₁-C₆)-alkoxy, 2-Tetrahydrofuranylmethoxy, 2-Tetrahydropyranylmethoxy, Ethoxyethoxyethoxy,Methoxyethoxyethoxy oder Cyclopropylmethoxy;
R² bedeutet Halogen;
R³ bedeutet (C₁-C₆)-Alkylsulfonyl;
R¹² bedeutet Wasserstoff oder Benzoyl und
Z bedeutet CH₂, CH(CH₃) oder C(CH₃)₂.

2. Benzoylcyclohexandione nach Anspruch 1, worin
R¹ bedeutet Halogen-(C₁-C₆)-alkoxy, (C₃-C₈)-Cycloalkoxy, Benzylethoxy, 2-Tetrahydrofuranylmethoxy, 2-Tetrahydropyranylmethoxy, Ethoxyethoxyethoxy, Methoxyethoxyethoxy oder Cyclopropylmethoxy;
R² bedeutet Chlor,
R³ bedeutet Methylsulfonyl;
R¹² bedeutet Wasserstoff, und
Z bedeutet CH₂.

3. Benzoylcyclohexandione nach Anspruch 2, worin
R¹ 2,2,2-Trifluorethoxy, 2,2-Difluorethoxy, 3,3,3,2,2-Pentafluorpropoxy, Cyclopentoxy, Cyclobutyloxy, Cyclopropylmethoxy, Benzylethoxy, 2-Tetrahydrofuranylmethoxy, 2-Tetrahydropyranylmethoxy, Ethoxyethoxyethoxy, Methoxyethoxyethoxy oder Cyclopropylmethoxy bedeutet.

4. Benzoylcyclohexandione nach Anspruch 2, worin
R¹ 2,2,2-Trifluorethoxy, 2,2-Difluorethoxy, 3,3,3,2,2-Pentafluorpropoxy, Benzylethoxy, 2-Tetrahydrofuranylmethoxy, 2-Tetrahydropyranylmethoxy, Ethoxyethoxyethoxy oder Methoxyethoxyethoxy bedeutet.

5. Herbizide Mittel, **gekennzeichnet durch** einen herbizid wirksamen Gehalt an mindestens einer Verbindung der allgemeinen Formel (I) gemäß einem der Ansprüche 1 bis 4.

6. Herbizide Mittel nach Anspruch 5 in Mischung mit Formulierungshilfsmitteln.

7. Verfahren zur Bekämpfung unerwünschter Pflanzen, **dadurch gekennzeichnet, daß** man eine wirksame Menge mindestens einer Verbindung der allgemeinen Formel (I) gemäß einem der Ansprüche 1 bis 4 oder eines herbiziden Mittels nach Anspruch 5 oder 6 auf die Pflanzen oder auf den Ort des unerwünschten Pflanzenwachstums appliziert.

8. Verwendung von Verbindungen der allgemeinen Formel (I) nach einem der Ansprüche 1 bis 3 oder von herbiziden Mitteln nach Anspruch 5 oder 6 zur Bekämpfung unerwünschter Pflanzen.

9. Verwendung nach Anspruch 8, **dadurch gekennzeichnet, daß** die Verbindungen der allgemeinen Formel (I) zur Bekämpfung unerwünschter Pflanzen in Kulturen von Nutzpflanzen eingesetzt werden.

10. Verwendung nach Anspruch 9 **dadurch gekennzeichnet, daß** die Nutzpflanzen transgene Nutzpflanzen sind.

## Claims

1. Benzoylcyclohexanediones of the general formula (I) 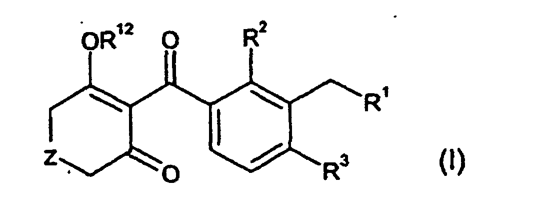 wherein
R¹ is halo-(C₁-C₆)-alkoxy, (C₃-C₈)-cycloalkoxy, aryl-(C₁-C₆)-alkoxy, 2-tetrahydrofuranylmethoxy, 2-tetrahydropyranylmethoxy, ethoxyethoxyethoxy, methoxyethoxyethoxy or cyclopropylmethoxy;
R² is halogen;
R³ is (C₁-C₆)-alkylsulfonyl;
R¹² is hydrogen or benzoyl; and
Z is CH₂, CH(CH₃) or C(CH₃)₂.

2. Benzoylcyclohexanediones according to claim 1, wherein
R¹ is halo-(C₁-C₆)-alkoxy, (C₃-C₈)-cycloalkoxy, benzylethoxy, 2-tetrahydrofuranylmethoxy, 2-tetrahydropyranylmethoxy, ethoxyethoxyethoxy, methoxyethoxyethoxy or cyclopropylmethoxy;
R² is chlorine;
R³ is methylsulfonyl;
R¹² is hydrogen; and
Z is CH₂.

3. Benzoylcyclohexanediones according to claim 2, wherein
R¹ is 2,2,2-trifluoroethoxy, 2,2-difluoroethoxy, 3,3,3,2,2-pentafluoropropoxy, cyclopentoxy, cyclobutyloxy, cyclopropylmethoxy, benzylethoxy, 2-tetrahydrofuranylmethoxy, 2-tetrahydropyranylmethoxy, ethoxyethoxyethoxy, methoxyethoxyethoxy or cyclopropylmethoxy.

4. Benzoylcyclohexanediones according to claim 2, wherein
R¹ is 2,2,2-trifluoroethoxy, 2,2-difluoroethoxy, 3,3,3,2,2-pentafluoropropoxy, benzylethoxy, 2-tetrahydrofuranylmethoxy, 2-tetrahydropyranylmethoxy, ethoxyethoxyethoxy or methoxyethoxyethoxy.

5. Herbicidal compositions **characterized by** a herbicidally active content of at least one compound of the general formula (I) as per any one of claims 1 to 4.

6. Herbicidal compositions according to claim 5 in admixture with formulation auxiliaries.

7. A process for controlling undesired plants, **characterized in that** an effective amount of at least one compound of the general formula (I) as per any one of claims 1 to 4 or of a herbicidal composition according to claim 5 or 6 is applied to the plants or to the site of the undesired plant growth.

8. Use of compounds of the general formula (I) according to any one of claims 1 to 3 or of herbicidal compositions according to claim 5 or 6 for controlling undesired plants.

9. Use according to claim 8, **characterized in that** the compounds of the general formula (I) are employed for controlling undesired plants in crops of useful plants.

10. Use according to claim 9, **characterized in that** the useful plants are transgenic useful plants.

## Revendications

1. Benzoylcyclohexandiones de formule générale (I) 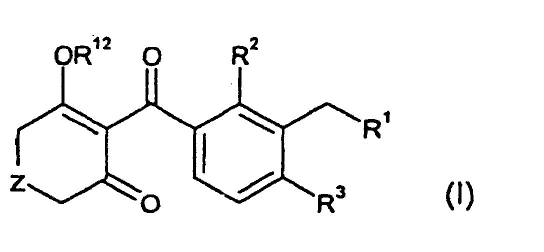 où
R¹ représente un groupe halogénoalkoxy en C₁-C₆, cycloalkoxy en C₃-C₈, arylalkoxy en C₁-C₆, 2-tétrahydrofuranylméthoxy, 2-tétrahydropyranylméthoxy, éthoxyéthoxyéthoxy, méthoxyéthoxyéthoxy ou cyclopropylméthoxy ;
R² représente un atome d'halogène ;
R³ représente un groupe (alkyl en C₁-C₆)sulfonyle ;
R¹² représente un atome d'hydrogène ou un groupe benzoyle et
Z représente un groupe CH₂, CH(CH₃) ou C(CH₃)₂.

2. Benzoylcyclohexanediones selon la revendication 1, où
R¹ représente un groupe halogénoalkoxy en C₁-C₆, cycloalkoxy en C₃-C₈, benzyléthoxy, 2-tétrahydrofuranylméthoxy, 2-tétrahydropyranylméthoxy, éthoxyéthoxyéthoxy, méthoxyéthoxyéthoxy ou cyclopropylméthoxy ;
R² représente un atome de chlore ;
R³ représente un groupe méthylsulfonyle ;
R¹² représente un atome d'hydrogène, et
Z représente un groupe CH₂.

3. Benzoylcyclohexanediones selon la revendication 2, où
R¹ représente un groupe 2,2,2-trifluoroéthoxy, 2,2-difluoroéthoxy, 3,3,3,2,2-pentafluoropropoxy, cyclopentoxy, cyclobutyloxy, cyclopropylméthoxy, benzyléthoxy, 2-tétrahydrofuranylméthoxy, 2-tétrahydropyranylméthoxy, éthoxyéthoxyéthoxy, méthoxyéthoxyéthoxy ou cyclopropylméthoxy.

4. Benzoylcyclohexanediones selon la revendication 2, où
R¹ représente un groupe 2,2,2-trifluoroéthoxy, 2,2-difluoroéthoxy, 3,3,3,2,2-pentafluoropropoxy, benzyléthoxy, 2-tétrahydrofuranylméthoxy, 2-tétrahydropyranylméthoxy, éthoxyéthoxyéthoxy ou méthoxyéthoxyéthoxy.

5. Agents herbicides **caractérisés par** une teneur efficace herbicide en au moins un composé de formule générale (I) selon l'une des revendications 1 à 4.

6. Agents herbicides selon la revendication 5 en mélange avec des adjuvants de formulation.

7. Procédé pour la lutte contre une végétation indésirable, **caractérisé en ce qu'**on applique une quantité efficace d'au moins un composé de formule générale (I) selon l'une des revendications 1 à 4 ou d'un agent herbicide selon la revendication 5 ou 6 aux plantes ou à l'endroit de pousse de la végétation indésirable.

8. Utilisation de composés de formule générale (I) selon l'une des revendications 1 à 3 ou d'agents herbicides selon la revendication 5 ou 6 pour la lutte contre la végétation indésirable.

9. Utilisation selon la revendication 8, **caractérisée en ce qu'**on utilise les composés de formule générale (I) dans la lutte contre une végétation indésirable dans les cultures de plantes utiles.

10. Utilisation selon la revendication 9, **caractérisée en ce que** les plantes utiles sont des plantes transgéniques.
